# EUROPÄISCHE PATENTANMELDUNG

(11) **EP 0 814 059 A1**
(43) Veröffentlichungstag der Anmeldung: **29.12.1997**
(21) Anmeldenummer: 97109945.2
(22) Anmeldetag: 18.06.1997
(51) Int. Cl.: C01B 37/02, B01J 37/03, C12N 11/14, A61K 7/02

(54) **Mesoporöse Oxidformkörper**

(30) Priorität: 21.06.1996 DE 19624862
(71) Anmelder: BASF AKTIENGESELLSCHAFT, 67056 Ludwigshafen (DE)
(72) Erfinder: Müller, Ulrich, Dr., 67434 Neustadt (DE); Schacht, Stefan, 65191 Wiesbaden (DE); Schüth, Ferdi, Prof., 61400 Oberursel (DE); Quisheng, Huo, Goleta, California 93117 (US); Stucky, Galen, Prof., Goleta, California 93117 (US)
(74) Vertreter: Isenbruck, Günter, Dr.

(57) **Zusammenfassung**

In einem Verfahren zur Herstellung mesoporöser Oxidformkörper werden Oxidvorläufer in einem Reaktionsmedium aus mindestens zwei nicht mischbaren Fluiden in Gegenwart von Tensiden umgesetzt.

## Beschreibung

Die Erfindung betrifft mesoporöse Oxidformkörper, die herstellbar sind durch Umsetzung von Oxidvorläufern in einem Reaktionsmedium aus mindestens zwei nicht mischbaren Fluiden in Gegenwart von Tensiden.

Aus der WO 91/11390 sind mesoporöse Molekularsiebe mit einer den Silikaten und Alumosilikaten verwandten chemischen Zusammensetzung bekannt. Die Synthese dieser Materialien mit der Bezeichnung MCM-41 erfolgt durch Umsetzung von Metallsalzen mit kationischen Tensiden, wie etwa Hexadecyltrimethylammonium in Form flüssigkristalliner Anordnungen, wie Schablonen, bei der Kondensation des Silikatgerüstes.

Die Stoffe weisen eine Kombination aus hoher Festkörperoberfläche, großem Porenvolumen und einer regelmäßigen Anordnung des Silikat- oder Alumosilikatgerüstes zu Poren im Bereich der Mesoporen von etwa 1,5 bis etwa 10 nm auf. Damit liegen regelmäßig strukturierte Porenöffnungsweiten vor, die deutlich größer sind als diejenigen von beispielsweise bekannten Zeolithen oder zeolithanalogen Alumophosphaten (Atlas of Zeolite Structure Types, Butterworths, 1978 und J. Am. Chem. Soc. 114 (1992) 10834 bis 10843).

Als Anwendungen der mesoporösen Molekularsiebe werden die katalytische Umwandlung von Kohlenwasserstoffen in Crack- oder Hydrocrackreaktionen und in der Umwandlung von Stickoxiden, sowie zur selektiven Adsorption von Komponenten aus Mischungen genannt.

Aus der EP-A-0 670 286 sind mesoporöse Molekularsiebe bekannt, die auf einer Vielzahl von Metalloxiden basieren können, beispielsweise auf Eisenoxid oder Bleioxid. Die Molekularsiebe werden hergestellt durch Kristallisation von Mischungen aus anionischen Tensiden und Isopoly- oder Heteropolykationen der Metalloxide in wässrigen Systemen. Als anionisches Tensid wird beispielsweise das Natriumsalz der Hexadecylsulfonsäure verwendet.

Ein Nachteil der bekannten Verfahren besteht darin, daß die erhaltenen Molekularsiebe vielfach in Form von feinstteiligen Pulvern anfallen. Für viele Anwendungen, beispielsweise als heterogene Katalysatoren, müssen daher auf die Synthese Formgebungsschritte folgen, wobei durch Binderzusatz Formkörper als Stränge, Extrudate verschiedener Geometrien oder durch Aufbauagglomeration auch Kugeln in Größen von typischerweise 0,5 bis 6 mm hergestellt werden. Der Aufbau monolithischer Wabenkörper, wie sie etwa in der katalytischen Abgasbehandlung eingesetzt werden, ist entsprechend sehr aufwendig und erfordert eine mehrstufige Verfahrensführung und eine intensive Prozessüberwachung.

Eine Aufgabe der vorliegenden Erfindung ist die Bereitstellung eines Verfahrens zur Herstellung von mesoporösen Oxidformkörpern, bei dem es möglich ist, die Oxidformkörper gleich in der gewünschten makroskopischen Geometrie auszubilden. Insbesondere soll das Verfahren die Herstellung von Oxiden mit P6-Symmetrie bzw. P6_{3/mmc}-Symmetrie erlauben.

Eine weitere Aufgabe der vorliegenden Erfindung ist die Bereitstellung eines Verfahrens zur Herstellung von mesoporösen Oxidformkörpern, das die Herstellung sehr einheitlich strukturierter Oxidformkörper erlaubt.

Eine weitere Aufgabe der Erfindung ist die Bereitstellung der entsprechenden Oxidformkörper.

Die Aufgaben werden gelöst durch ein Verfahren zur Herstellung mesoporöser Oxidformkörper durch Umsetzung von Oxidvorläufern in einem Reaktionsmedium aus mindestens zwei nicht mischbaren Fluiden in Gegenwart von Tensiden. Demgemäß wurden die verbesserten mesoporösen Oxidformkörper erhalten, wie sie eingangs beschrieben wurden.

Es wurde gefunden, daß man die Aufgaben lösen und die vorstehend aufgeführten Nachteile vermeiden kann, wenn man die Synthese der mesoporösen Oxidformkörper an der Grenzfläche von zwei oder mehreren nicht mischbaren Fluiden durchführt. Dabei können mesoporöse bzw. mesostrukturierte Oxide in Form dünner Filme oder Schichten, wie auch von Pulvern oder Kugeln erhalten werden.

"Mesoporös" bedeutet dabei, daß Porendurchmesser zwischen 2 bis 50 nm erhalten werden.

Die Umsetzung kann in allen geeigneten Reaktionsmedien aus mindestens zwei nicht mischbaren Fluiden durchgeführt werden. Bei den Fluiden kann es sich um Flüssigkeiten oder Gase, wie auch um überkritische Fluide, wie etwa Kohlendioxid oder Ammoniak handeln. Die Umsetzung erfolgt dabei an der Flüssig/Flüssig- oder Flüssig/Gas-Grenzfläche.

Gemäß einer Ausführungsform der Erfindung besteht das Reaktionsmedium aus Wasser und mindestens einer nicht mit Wasser mischbaren Phase. Dabei kann die Wasserphase noch weitere Stoffe, beispielsweise Alkohole enthalten, so daß sie als wässrige Phase vorliegt.

Als nicht mit Wasser mischbare Phase kommen vorzugsweise organische Lösungsmittel in Betracht, die nicht mit Wasser mischbar sind. Insbesondere sind Lösungsmittel geeignet, die unter den Reaktionsbedingungen nicht mit der Umsetzung wechselwirken. Ein Beispiel eines geeigneten Lösungsmittels ist Mesitylen, oder auch aliphatische oder aromatische Kohlenwasserstoffe, etwa Hexan oder Toluol können eingesetzt werden.

### TENSIDE

Die Umsetzung von Oxidvorläufern erfolgt in Gegenwart von Tensiden.

Vorzugsweise wird das eingesetzte Tensid so gewählt, daß es an der Grenzfläche der Fluide vorliegt. Geeignete Tenside sind beispielsweise aus der EP-A-0 670 286 bekannt. Geeignet sind anionische, kationische, wie auch nicht ionische Tenside. Beispiele geeigneter anionischer Tenside sind Verbindungen der allgemeinen Formel R-X,
in der
- X: COOY, OSO₃Y, SO₃Y, OPO₃Y, PO₃Y, COOH, SO₄⁻ , SO₃⁻, PO₄²⁻, PO₃²⁻,
- Y: ein Element der Gruppe Ia oder IIa des Periodensystems der Elemente zum Ladungsausgleich,
- R: R¹, (CH₂)ₙ-R² oder CHR³R⁴,
- R¹: C₆- bis C₆₀-Alkyl,
- R²: NR⁵-CO-R¹, NR⁵-SO₂-CHR¹R¹, OR¹, SR¹, SO₂-R¹, OOC-R¹, C₇- bis C₆₀-Alkylaryl, oder C₇- bis C₆₀-Alkylaryloxy,
- R³, R⁴: unabhängig voneinander C₆- bis C₆₀-Alkyl, COOR¹ oder CH₂-COOR¹,
- R⁵: Wasserstoff oder C₁- bis C₄-Alkyl und
- n: eine ganze Zahl von 0 bis 12 bedeuten.

Vorzugszweise stehen Y für Natrium oder Kalium, der Rest R¹ für C₈₋₅₀-Alkyl, besonders bevorzugt C₉₋₄₅-Alkyl, R² für C₁₀₋₅₀-Alkylaryl, besonders bevorzugt C₁₂₋₄₅-Alkylaryl oder C₁₀₋₅₀-Alkylaryloxy, besonders bevorzugt C₁₂₋₄₅-Alkylaryloxy, R³ und R⁴ unabhängig voneinander für C₈₋₅₀-Alkylreste, besonders bevorzugt C₉₋₄₅-Alkylreste, R⁵ für Methyl oder Ethyl, besonders bevorzugt für Methyl

Bevorzugt eingesetzt werden Alkalisalze von C₈₋₃₀-Alkylsulfonsäuren, besonders bevorzugt das Natriumsalz der Hexadecylsulfonsäure. Weiterhin bevorzugt eingesetzt werden C₈₋₃₀-Alkyltrimethylammoniumhalogenide, insbesondere Hexadecyltrimethylammoniumbromid, Octadecyltrimethylammoniumbromid oder Eicosyltrimethylammoniumbromid.

Beispiele verwendbarer anionischer Tenside sind carboxymethylierte Oxethylate oder Derivate von Aminosäuren, Sulfonate, wie Ligninsulfonate, Alkylbenzolsulfonate, Alkylnaphthalinsulfonate, Alkylsulfonate, Olefinsulfonate, Sulfofettsäureester, Sulfofettsäureamide, Sulfobernsteinsäureester, Alkoxyalkylsulfonate, Acyloxyalkylsulfonate oder Acylaminoalkylsulfonate, Sulfate, wie Alkylsulfate oder Ethersulfate, Phosphonate oder Phosphate.

Das Tensid kann je nach Polarität sowohl in der wässrigen als auch in der organischen Phase, sowie in beiden Phasen enthalten sein. Das Tensid wirkt als Strukturbildner bzw. Templat, da es die Bildung der mesoporösen Oxidformkörper an der Grenzfläche der Fluide bewirkt.

### OXIDVORLÄUFER

Der erfindungsgemäß eingesetzte Oxidvorläufer ist gemäß einer Ausführungsform der Erfindung eine Verbindung der Elemente Si, Al, B, Ge, der Elemente der Gruppen IIIa, IIb, IVb, Vb, VIb des Periodensystems der Elemente, Be, Sn, Pb, Bi, Cu, Fe Co, Ni, Ce, Mn. Die Oxidvorläufer liegen dabei vorzugsweise in Form von Salzen oder in Form von hydrolysierbaren (metall)organischen Verbindungen vor. Der Oxidvorläufer kann somit der organischen Phase zugesetzt werden, beispielsweise als metallorganische Komponente, wie als Alkoholat, Grignardverbindung, Alkylat, Chelat mit Acetonylacetat. Der Oxidvorläufer kann auch in der wässrigen Phase eingesetzt werden, beispielsweise in Form löslicher Salze oder Kolloide, wie der Sulfate, Phosphate, Nitrate, Carbonate, Halogenide oder Perchlorate, vorzugsweise Sulfate, Phosphate und Nitrate, besonders bevorzugt Sulfate und Nitrate.

Vorzugsweise wird der Oxidvorläufer in Form eines Alkoxylats eingesetzt, beispielsweise bei Verwendung von Silicium als Metallatom in Form von Tetraethoxysilan.

Vorzugsweise werden Verbindungen der Elemente Silicium, Aluminium, Bor, Titan, Vanadium, Zirkon, Zinn oder Gemische davon verwendet, die vorzugsweise in Form von Methoxy- oder Ethoxyverbindungen vorliegen.

### HERSTELLUNGSVERFAHREN

Oxidvorläufer und Tensid werden in eines oder mehrere der nicht mischbaren Fluide eingetragen. Bei der Verwendung von Wasser und mindestens einem nicht mit Wasser mischbaren organischen Lösungsmittel wird das Tensid vorzugsweise in die Wasserphase bzw. wässrige Phase (die beispielsweise noch Alkohol enthalten kann) eingetragen, der Oxidvorläufer je nach Art der Verbindung in die Wasserphase bzw. die organische Phase. Vorzugsweise wird der Oxidvorläufer als Alkoxyverbindung, besonders bevorzugt C₁₋₄-Alkoxyverbindung, insbesondere Ethoxyverbindung im organischen Lösungsmittel vorgelegt.

An der Phasengrenze der nicht mischbaren Fluide bildet sich dabei der mesoporöse Oxidformkörper. Wird die Phasengrenze nicht bewegt, so fällt der Oxidformkörper in Form eines Films oder einer Schicht an, deren Dicke mit zunehmender Umsetzungszeit zunimmt. Gemäß einer Ausführungsform der Erfindung sind dabei Dicken von 3 bis 3000 µm, vorzugsweise 10 bis 1000 µm, insbesondere etwa 500 µm erreichbar.

Wird das Reaktionsmedium während der Umsetzung durchmischt, beispielsweise durch starkes Rühren, so bildet sich eine Emulsion der einen fluiden Phase in der anderen fluiden Phase aus. Bei Verwendung von Wasser und mindestens einem nicht mit Wasser mischbaren organischen Lösungsmittel bildet sich somit eine Emulsion entweder des Lösungsmittels in Wasser oder von Wasser im Lösungsmittel aus. Vorzugsweise wird eine Emulsion des Lösungsmittels in Wasser verwendet. Dabei können sphärische Partikel hergestellt werden, da die Entstehung der Oxidformkörper an der nun kugelförmigen Oberfläche stattfindet. Die sphärischen Partikel liegen zumindest teilweise in Form von Hohlkugeln vor. Durch Variation der Mischintensität bzw. der Rührergeschwindigkeit bei mechanischem Rühren kann die Größe der Kugeln bzw. der Hohlkugeln eingestellt werden. Das erfindungsgemäße Verfahren erlaubt dabei die Herstellung von sphärischen Formkörpern mit einem sehr engen Größenverteilungsbereich.

Bringt man gemäß einer Ausführungsform der Erfindung einen Träger in den Bereich der Grenzschicht zwischen den unterschiedlichen Phasen ein, so kann eine Abscheidung des mesoporösen Oxidformkörpers auf diesem Träger erfolgen. Beispielsweise kann der Träger ein inerter poröser Träger sein, wie ein poröses Glas, Aluminiumoxid, Kieselgel oder Tonerde, Keramik, Metall oder Metallpackung, wie sie beispielsweise für statische Mischer eingesetzt wird. Der inerte Träger kann somit mit dem mesoporösen Oxidformkörper belegt oder imprägniert werden, wodurch Komposit-Materialien entstehen, die eine gute mechanische Stabilität aufweisen, insbesondere im Vergleich zu trägerfreien mesoporösen Oxidformkörpern.

Die wässrige wie auch die organische Phase können weitere Zusätze enthalten, wie Metall- oder Edelmetallionen, vorzugsweise der Gruppe VIIIb des Periodensystems der Elemente. Dadurch ist es möglich, katalytisch wirksame Metalle in den mesoporösen Oxidformkörper einzubringen, etwa bei Anwendungen als Katalysator. Es können ebenso für biotechnologische Anwendungen beispielsweise Enzyme in die wässrige Phase eingebracht werden, die dann mit einkondensiert werden. Auch der Einschluß von pharmazeutischen Wirkstoffen bzw. pharmakologisch wirksamen Verbindungen ist möglich aufgrund der milden Reaktionsbedingungen. Somit können mesoporöse Oxidformkörper hergestellt werden, die einen pharmazeutischen Wirkstoff bei einer bestimmten Applikation retardiert freisetzen.

Die Umsetzung erfolgt gemäß einer Ausführungsform der Erfindung bei einer Temperatur von minus 10 bis 150 °C, vorzugsweise 10 bis 90 °C, besonders bevorzugt 20 bis 65 °C. Die Umsetzung kann bei Atmosphärendruck, Unterdruck oder Überdruck ausgeführt werden, gemäß einer Ausführungsform der Erfindung bei einem Druck von 0,4 bis 300 bar. Vorzugsweise wird sie bei Atmosphärendruck ausgeführt.

Das erfindungsgemäße Verfahren kann sowohl im basischen als auch im sauren oder neutralen pH-Bereich durchgeführt werden. Bei der Herstellung von SiO₂-Oxidformkörpern ist ein saurer pH-Bereich bevorzugt, vorzugsweise von pH 5 bis pH 1.

Die erfindungsgemäß hergestellten mesoporösen Oxidformkörper weisen gemäß einer Ausführungsform der Erfindung Poren im Größenbereich von 1 bis 20 nm, vorzugsweise 2 bis 8 nm und insbesondere 2,5 bis 4,5 nm auf.

Dabei liegen gemäß einer Ausführungsform der Erfindung die spezifischen Oberflächen der mesoporösen Oxidformkörper bei 100 bis 1500 m²/g, vorzugsweise bei 200 bis 1200 m²/g, insbesondere bei 300 bis 800 m²/g. Die spezifischen Oberflächen werden dabei mittels Tieftemperatur-Stickstoffadsorption bestimmt und nach Langmuir ausgewertet.

Die erfindungsgemäßen mesoporösen Oxidformkörper weisen gemäß einer Ausführungsform der Erfindung eine P6 oder eine P6_{3/mmc}-Symmetrie auf, wie durch röntgenographische Analyse durch Auswertung der Reflexe hervorgeht.

Zur Herstellung einer P6_{3/mmc}-Symmetrie im mesoporösen Oxidformkörper wird gemäß einer Ausführungsform der Erfindung ein geminales Tensid verwendet, wie es beispielsweise beschrieben ist in Q. Hoo, R. Leon, P.M. Petroff, G.D. Stucky, Science 268, 1324 (1995).

Der erfindungsgemäße Formkörper kann nach der Herstellung von überschüssigem Lösungsmittel oder Tensid durch Erhitzen auf eine Temperatur von etwa 350 bis 650°C, vorzugsweise etwa 500°C befreit werden. Dabei wird der Formkörper gleichzeitig kalziniert.

### VERWENDUNG DER MESOPORÖSEN OXIDFORMKÖRPER

Die erfindungsgemäßen mesoporösen Oxidformkörper können in einer Vielzahl von Anwendungen eingesetzt werden. Die bei der Synthese in einem unbewegten Reaktionsmedium entstehenden Oxidfilme oder Schichten können als selbsttragende Membranen verwendet werden, beispielsweise in Membran-Trenn- und Reinigungsverfahren. Die Membranen können ebenso für katalytische Umsetzungen in Membranreaktoren oder bei Reaktivdestillationen verwendet werden, bei denen eine katalytische Umsetzung mit einer Destillation verbunden wird. Ebenso ist die Verwendung zur Informationsspeicherung möglich, wie auch die Verwendung für elektronische, optische oder elektrooptische Geräte.

Die erfindungsgemäßen mesoporösen Oxidformkörper können darüber hinaus als Katalysatoren verwendet werden. Beispiele geeigneter katalytischer Umsetzungen sind die Oxifunktionalisierung von Kohlenwasserstoffen, die Oxidation von Olefinen zu Oxiranen, Aromatenalkylierungen, Hydrierungen, Dehydrierungen, Hydratisierungen, Dehydratisierungen, Isomerisierungen, Additions- und Eliminierungsreaktionen, nukleophile und elektrophile Substitutionsreaktionen, Dehydrocyclisierungen, Hydroxylierungen von Heteroaromaten und Aromaten, Epoxid-Aldehyd-Umlagerungen, Aminierungen von monomeren und oligomeren Olefinen, Kondensationsreaktionen vom Aldoltyp, Polymerisationsreaktionen, Veresterungen und Veretherungsreaktionen, wie auch katalytische Umsetzungen von Abgasen und Rauchgasen bzw. Stickoxidentfernung. Die Verwendungen zur Aufnahme und verzögerten Freisetzung von Arzneimitteln wie auch zur Aufnahme von Pigmenten, die dann in dem mesoporösen Oxidkörper verkapselt vorliegen, ist bereits vorstehend beschrieben.

Auch eine Verwendung als Sorbentien sowie zur Herstellung von Oxidkeramiken ist möglich, wie auch die Verwendung bei der Stofftrennung.

Erfindungsgemäße mesoporöse Oxidformkörper aus Siliciumdioxid sind in den Figuren dargestellt.

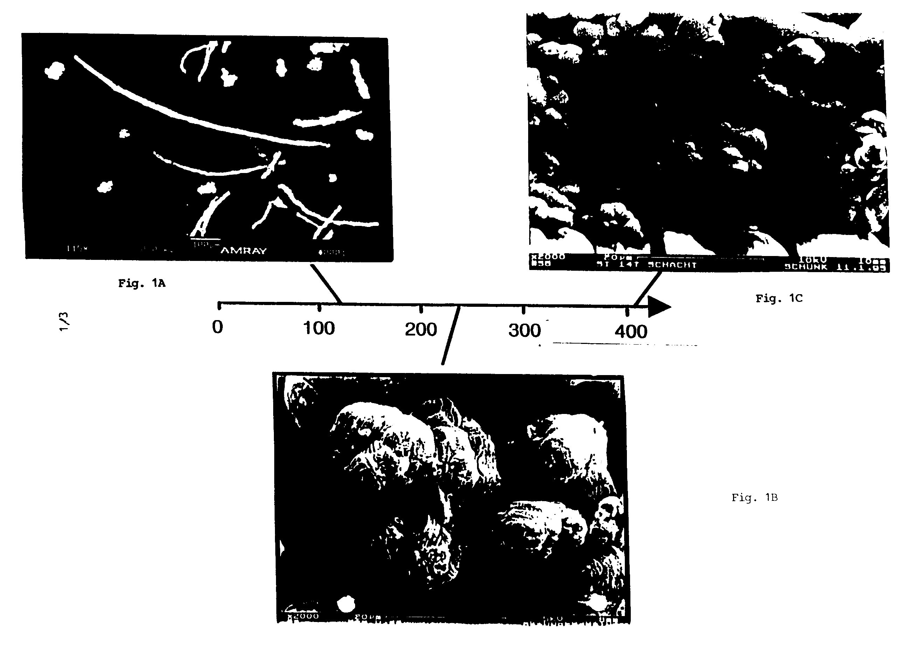

Dabei zeigt Figur 1 die Abhängigkeit der Morphologie der mesoporösen Oxidformkörper von der Rührgeschwindigkeit, die in U/min angegebenen ist. Bei niedrigen Rührgeschwindigkeiten (A) werden faserartige Teilchen gebildet, während bei höheren Rührgeschwindigkeiten (B, C) sphärische Teilchen entstehen. Die Größe der Teilchen nimmt mit zunehmender Rührgeschwindigkeit ab. Die Darstellungen sind SEM (Scanning Electron Microscopy) Aufnahmen der erfindungsgemäßen mesoporösen Oxidformkörper. Der angegebene Balken in den Abbildungen hat eine Länge von 20 µm bzw. 100 µm für die Abbildung bei einer Rührgeschwindigkeit von etwa 120 U/min.

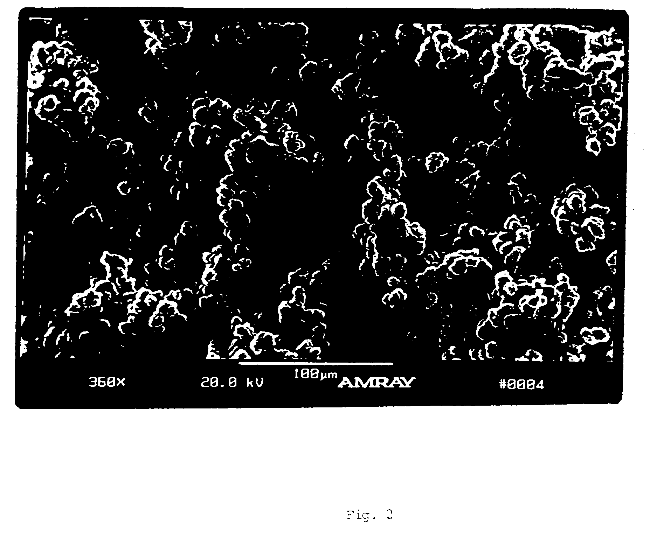

Figur 2 zeigt eine SEM-(Scanning Electron Microscopy)-Abbildung eines mesoporösen Oxidformkörpers aus Siliciumdioxid, wobei die kugelförmigen Teilchen agglomeriert sind.

Figur 3 zeigt eine SEM-Abbildung einer Hohlkugel aus Siliciumdioxid, die aus agglomerierten plattenartigen Teilchen besteht, die die P6_{3/mmc}-Struktur aufweisen.

Die Erfindung wird nachstehend durch Beispiele erläutert.

### BEISPIEL 1

Aus 4,74 g eines Tensids der Zusammensetzung C₁₆-Alkyl-N(CH₃)₃Br gelöst in 250 ml deionisiertem Wasser wird unter Eintragen von 34 ml einer wässrigen HCl (37 Gew.-%) eine wässrige Phase hergestellt.

Zu dieser Phase gibt man 7 g Mesitylen und 4 g Tetraethoxysilan (vetrieben von Aldrich) innerhalb von 30 Minuten unter langsamem Rühren. Dabei bildet sich eine Emulsion.

80 ml dieser Emulsion rührt man für weitere 120 Min. bei 45°C mit einer Drehzahl von 120 U/min. Das Endprodukt besteht aus faserförmigem mesoporigem SiO₂. Die spezifische Oberfläche beträgt dabei 1400 m²/g.

Weitere 80 ml dieser Emulsion rührt man für weitere 120 Minuten bei 45°C, jedoch mit einer Drehzahl von 250 U/min. Man erhält als Endprodukt mesoporiges SiO₂ in spherischer Form mit Partikeldurchmessern von etwa 15 bis 20 µm.

Weitere 80 ml dieser Emulsion rührt man für 120 Minuten bei 45°C mit einer Drehzahl von 410 U/min. Man erhält als Endprodukt ebenfalls mesoporiges SiO₂ mit Partikeldurchmessern von 2 bis 5 µm.

Tetraethoxysilan wird dabei an der Öl/Wasser-Grenzfläche hydrolisiert und bildet dabei den mesoporösen Siliciumdioxidformkörper. Die Form entspricht dabei der Tropfenform der durch Rühren erzeugten Öl-in-Wasser-Emulsion, da der Aufbau des Formkörpers an der Phasengrenze erfolgt.

SEM-Aufnahmen der erhaltenen drei mesoporösen Oxidformkörpern sind in Figur 1 dargestellt. Sie zeigen, daß durch die Wahl der Rührbedingungen die makroskopische Form des entstandenen mesoporösen SiO₂ gesteuert werden kann.

### BEISPIEL 2

Aus 4,74 g eines Tensids der Zusammensetzung C₁₆-Alkyl-N(CH₃)₃Br, gelöst in 250 ml deionisiertem Wasser stellt man mit 34 ml einer wässrigen HCl (37 Gew.-%) eine wässrige Lösung her.

Diese Lösung gibt man ohne Rühren vorsichtig in einer Kristallisationsschale mit einem Durchmesser von 10 cm auf die Oberfläche von darin enthaltenen 250 ml Tribrommethan. Nach etwa 10 Minuten dosiert man mit einer Spritze 4 g Tetraethoxysilan (Aldrich) langsam an der Phasengrenzfläche hinzu, ohne dabei wässrige und organische Phase zu durchmischen.

Innerhalb von 5 bis 10 min nach der Zugabe des Tetraethoxysilans bildet sich an der Phasengrenzfläche zunächst ein Film, der allmählich zu einer Feststoffschicht aus mesoporösem SiO₂ heranwächst. Der Durchmesser der so erhaltenen Feststoffscheibe ist etwa 10 cm. Die Dicke betrug zwischen 0,5 und 1 mm.

Bei der röntgenographischen Analyse zeigt der so erhaltene Feststoff typische Peaks bei 19,8 und 35,2° 2Theta.

### BEISPIEL 3

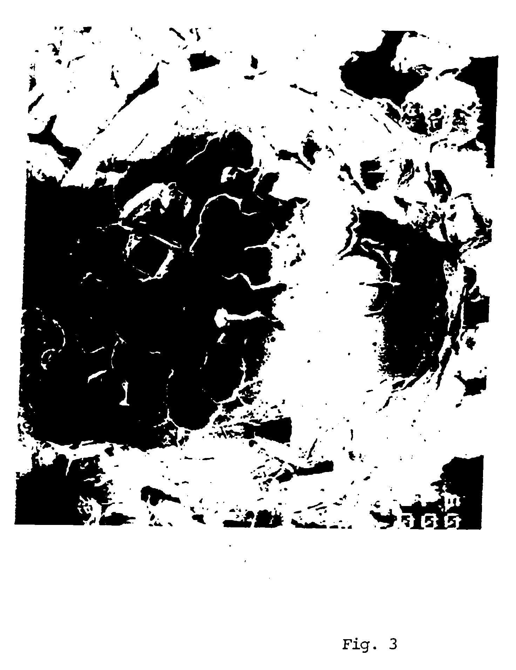

1 g eines geminalen Tensids (18-6-1, beschrieben in Q. Huo, R. Leon, P.M. Petroff, G.D. Stucky, Science 268, 1324 (1995)) wurden in 56 g von 1,5 M HCl gelöst. Sodann wurden 4 g Tetraethoxysilan in 1,2 g Mesitylen innerhalb von 40 Minuten unter Rühren bei Raumtemperatur eingetragen. Nach weiterem Rühren für 3 Stunden bei der Temperatur entstand ein weißer Festkörper. Der Festkörper enthält Hohlkugeln von etwa 50 µm Durchmesser und besteht aus ineinandergewachsenen plattenartigen Teilchen, die der P6_{3/mmc}-Phase entsprechen. Das Röntgenstreuungsmuster ist identisch mit dem, was für die P6_{3/mmc}-Phase beschrieben ist (a = 5,02 nm, c = 8,84 nm, c/a = 1,63 nach Kalzinieren bei 500°C). Ein entsprechendes Teilchen ist in Abbildung 3 dargestellt.

## Patentansprüche

1. Verfahren zur Herstellung mesoporöser Oxidformkörper durch Umsetzung von Oxidvorläufern in einem Reaktionsmedium aus mindestens zwei nicht mischbaren Fluiden in Gegenwart von Tensiden.

2. Verfahren nach Anspruch 1, wobei das Reaktionsmedium aus Wasser und mindestens einem nicht mit Wasser mischbaren organischen Lösungsmittel besteht.

3. Verfahren nach Anspruch 1 oder 2, wobei die Oxidvorläufer Verbindungen der Elemente Si, Al, B, Ge, der Elemente der Gruppen IIIa, IIb, IVb, Vb, VIb des Periodensystems der Elemente, Be, Sn, Pb, Bi, Cu, Fe, Co, Ni, Ce, Mn oder Gemische davon sind und die Oxidvorläufer in Form von Salzen oder hydrolysierbaren (metall)organischen Verbindungen vorliegen.

4. Verfahren nach einem der vorstehenden Ansprüche, wobei das Tensid so gewählt ist, daß es an der Grenzfläche der Fluide vorliegt, vorzugsweise ausgewählt aus Verbindungen der allgemeinen Formel R-X,
in der
X COOY, OSO₃Y, SO₃Y, OPO₃Y, PO₃Y, COOH, SO₄⁻, SO₃⁻, PO₄²⁻ and PO₃²⁻,
Y ein Element der Gruppe Ia oder IIa des Periodensystems der Elemente zum Ladungsausgleich,
R R¹, (CH₂)ₙ-R² oder CHR³R⁴,
R¹ C₆- bis C₆₀-Alkyl,
R² NR⁵-CO-R¹, NR⁵-SO₂-CHR¹R¹, OR¹, SR¹, SO₂-R¹, OOC-R¹, C₇- bis C₆₀-Alkylaryl, oder C₇- bis C₆₀-Alkylaryloxy,
R³, R⁴ unabhängig voneinander C₆- bis C₆₀-Alkyl, COOR¹ oder CH₂COOR¹,
R⁵ Wasserstoff oder C₁- bis C₄-Alkyl und
n eine ganze Zahl von 0 bis 12 bedeuten.

5. Verfahren nach einem der vorstehenden Ansprüche, wobei im Reaktionsmedium zusätzlich Metalle der Gruppe VIIIb des Periodensystems der Elemente oder pharmazeutische Wirkstoffe oder Enzyme oder Pigmente enthalten sind.

6. Verfahren nach einem der vorstehenden Ansprüche, wobei das Reaktionsmedium während der Umsetzung durchmischt wird.

7. Verfahren nach einem der Ansprüche 1 bis 5, wobei bei der Umsetzung ein Träger in den Bereich der Grenzschicht zwischen den nicht mischbaren Fluiden eingebracht wird.

8. Mesoporöser Oxidformkörper, herstellbar nach einem Verfahren, wie es in einem der Ansprüche 1 bis 7 definiert ist, der eine hexagonale Einheitszelle und P6-Symmetrie oder P6_{3/mmc}-Symmetrie (bestimmt mittels Röntgendiffraktometrie) aufweisen kann.

9. Mesoporöser Oxidkörper nach Anspruch 8, zusätzlich enthaltend ein Metall der Gruppe VIIIb des Periodensystems der Elemente oder einen pharmazeutischen Wirkstoff oder ein Enzym oder ein Pigment.

10. Verwendung von mesoporösen Oxidkörpern nach Anspruch 8 oder 9 als Katalysatoren oder Träger für Katalysatoren oder Arzneimittel oder Farbstoffe.
